# EUROPEAN PATENT SPECIFICATION

(11) **EP 3 081 940 B1**
(45) Date of publication and mention of the grant of the patent: **03.07.2019**
(21) Application number: 16165685.5
(22) Date of filing: 15.04.2016
(51) Int. Cl.: G01N 33/68

(54) **DIAGNOSTIC TESTING FOR IMMUNOLOGIC FOOD SENSITIVITY IN DOGS**
DIAGNOSEPRÜFUNG AUF IMMUNOLOGISCHE LEBENSMITTELSENSITIVITÄT BEI HUNDEN
TEST DE DIAGNOSTIC D'UNE ALLERGIE ALIMENTAIRE IMMUNOLOGIQUE CHEZ DES CHIENS

(30) Priority: 17.04.2015 US 201562149076 P; 14.04.2016 US 201615098380 P
(43) Date of publication of application: 19.10.2016
(60) Divisional application: 19175311.0
(73) Proprietor: Fine, Kenneth Davin, Dallas, TX 75238 (US)
(72) Inventor: Fine, Kenneth Davin, Dallas, TX 75238 (US)
(74) Representative: Gill Jennings & Every LLP

(56) References cited:
- US-A1- 2001 036 639
- US-A1- 2002 131 893
- US-A1- 2013 183 692
- PERRIER C ET AL: "Allergen-specific antibody and cytokine responses, mast cell reactivity and intestinal permeability upon oral challenge of sensitized and tolerized mice", CLINICAL & EXPERIMENTAL ALLERGY : JOURNAL OF THE BRITISH SOCIETY FOR ALLERGY AND CLINICAL IMMUNOLOGY, WILEY INTERSCIENCE, UK, vol. 40, no. 1, 1 January 2010 (2010-01-01), pages 153-162, XP009191561, ISSN: 0954-7894

## Description

### TECHNICAL FIELD

The present invention relates to immunologic food sensitivity in dogs.

### BACKGROUND

Currently, immunologic food sensitivities (e.g., allergy and/or intolerance) in animals may be difficult to identify. For example, to identify food sensitivity, the animal may be placed on an elimination diet. However, since pet food today often includes many ingredients, an elimination diet may be difficult to follow for an animal and/or expensive. Furthermore, needless elimination diets (e.g., when food sensitivities are not present) may cause unintended calorie reduction, nutritional deficiencies, and/or unnecessarily add high costs to feeding one's pet.

In addition, since animals can not verbally communicate symptoms to their owners, food sensitivities may go unnoticed and/or undetected for long periods of time, and continue to cause immunological reactions, complicating co-illnesses, and possibly even premature death. Additionally, since there are no highly accurate diagnostic methods currently available to diagnose food sensitivity in domestic animals, certain diagnosis of this problem in the presence of overt symptoms in animals is not commonplace.

### SUMMARY

In various implementations, a noninvasive, veterinary diagnostic testing system may be utilized to identify one or more food sensitivities and/or facilitate identification of diseases and/or disorders. The veterinary diagnostic testing system may include a veterinary diagnostic test, fecal sample collection set (e.g., tools to transfer feces, specimen container, fecal sample collection device, etc.), reagents, and/or other components. A fecal sample from a dog may be obtained. The veterinary diagnostic test may be performed on the obtained fecal sample. One or more food sensitivities may be identified based on the performance of the veterinary diagnostic test on the fecal sample. In some implementations, one or more diseases or disorders associated with the identified food sensitivity may be identified.

In various implementations, immunologic food sensitivity may be diagnosed in veterinary applications in dogs. A fecal sample may be obtained from a dog. A determination may be made whether a testing portion of the obtained fecal sample includes IgA associated with a set of food sensitivities and/or IgA antibody associated with a set of food sensitivities. The testing portion includes at least a portion of the fecal sample. A determination may be made that at least one of a set of immunologic food sensitivities are present in the dog if a determination is made that IgA associated with a set of food sensitivities and/or IgA antibody associated with a set of food sensitivities is present in the testing portion of the fecal sample of the dog.

Implementations may include one or more of the following features. The IgA may include antigliadin IgA and the IgA antibody may include antitissue transglutaminase IgA antibody, and the set of food sensitivities may include sensitivity to gluten, in some implementations. In some implementations, the fecal sample may be concentrated, and the testing portion may be obtained from the concentrated fecal sample. The testing portion may include an undiluted portion of the fecal sample. In some implementations, the fecal sample may be stirred prior to obtaining the testing portion. In The fecal sample may be concentrated prior to obtaining the testing portion. Concentrating the fecal sample may include centrifuging the fecal sample, and removing a supernatant portion from the centrifuged fecal sample. The testing portion may be obtained from the supernatant portion. In some implementations, the fecal sample may be freeze-dried to a solid fecal material. The solid fecal material may be reconstituted with water to form a reconstituted fecal sample, and the testing portion may be obtained from the reconstituted solid fecal material. In some implementations, a solution comprising one or more flags may be applied to the fecal sample to obtain a flagged fecal sample. The testing portion may be obtained from the flagged fecal sample. In some implementations, a hydrating agent may be applied to the fecal sample prior to obtaining the testing portion. In some implementations, a determination may be made whether a blood sample of the dog includes IgA, IgE, and/or IgG associated with the set of food sensitivities. Determining whether a set of immunologic food sensitivities is present in the dog may be further based on whether a determination is made that IgA, IgE, and/or IgG associated with the set of food sensitivities is present in a blood sample of the dog. In some implementations, a determination may be made whether a second testing portion of the obtained fecal sample includes at least one of IgA associated with a second set of food sensitivities or IgA antibody associated with a second set of food sensitivities if a determination is made that the set of immunologic food sensitivities is present in the dog. The second testing portion may include a second portion of the fecal sample. The second set of food sensitivities may or may not include a portion of the first set of food sensitivities. The first set of food sensitivities includes gluten sensitivity and/or corn sensitivity.

In various implementations, a veterinary kit to identify food sensitivity may be provided. The kit may include a veterinary diagnostic test to identifying the presence of a first set of food sensitivities in a dog. The veterinary diagnostic test includes a substrate and a test region. The test region includes antibody binding agent(s) coupled to the substrate. At least one of the antibody binding agents are adapted to couple to one or more first antibodies associated with a first set of food sensitivities when one or more of the first antibodies is present in a fecal sample obtained from a dog.

Implementations may include one or more of the following features. The veterinary diagnostic test may include a stick test adapted to contact the fecal sample. One or more of the first antibodies, if present in the fecal sample, may transfer to the veterinary diagnostic test by the contact. The veterinary diagnostic test may include an absorbent region adapted to transfer a testing portion of a fecal sample of an dog from the fecal sample to the test region of the veterinary diagnostic test. The veterinary diagnostic test may include one or more flags adapted to indicate coupling of one or more first antibodies in a fecal sample with one or more of the antibody binding agents of the veterinary diagnostic test. A flag may indicate that at least one of the first set of food sensitivities is present in the dog associated with the fecal sample. The kit includes a fecal sample collection tool. The veterinary diagnostic test is integrated in the collection tool. The veterinary diagnostic test is positioned on the fecal sample collection tool such that the veterinary diagnostic test contacts the fecal sample. The fecal sample collection tool may include an opening to allow at least a portion of fluid from a fecal sample to drain from the fecal sample collection tool. The fecal sample collection tool may include a removable stop. The stop may be adapted to close an opening in the fecal sample collection tool, and when the stop is removed fluid and/or the fecal sample may be drained from the fecal sample collection tool via the opening.

In various implementations, a veterinary diagnostic test may be a home kit, a clinical kit (e.g., for use in a clinic and/or point of care setting, such as an animal hospital), or a laboratory kit. The home kit, the clinical kit, and/or the laboratory kit may allow identification of one or sets of food sensitivities.

The details of one or more implementations are set forth in the accompanying drawings and the description below. Other features, objects, and advantages of the implementations will be apparent from the description and drawings.

### BRIEF DESCRIPTION OF THE DRAWINGS

For a more complete understanding of this disclosure and its features, reference is now made to the following description, taken in conjunction with the accompanying drawings, in which:
Figure 1A illustrates an implementation of an example veterinary diagnostic test.
Figure 1B illustrates an implementation of the example veterinary diagnostic test illustrated in Figure 1A, during use.
Figure 2 illustrates an implementation of an example process for testing for food sensitivities.
Figure 3 illustrates an implementation of an example integrated fecal collection tool.
Figure 4A illustrates an implementation of an example integrated fecal collection tool.
Figure 4B illustrates an implementation of the example integrated fecal collection tool illustrated in Figure 4A in which the collar has been removed.

Like reference symbols in the various drawings indicate like elements.

### DETAILED DESCRIPTION

In various implementations, a diagnostic test for food sensitivities in dogs (e.g., domestic, agricultural, non-domesticated captured from the wild, and/or otherwise) may be provided. The diagnostic tests and methods of the invention are carried out *in vitro,* using sample material that has been obtained from a dog. Food sensitivities may include immunological reactions in a dog in response to ingesting, consuming, injecting, and/or contacting foods or portions thereof. The veterinary diagnostic test may be noninvasive. The veterinary diagnostic tests may be for use in a clinical (e.g., in office and/or point of care testing), laboratory, and/or home environment.

Allowing identification of immunological food sensitivities may help reduce animal care costs, improve animal quality of life, and/or provide a greater understanding of animal health (e.g., for research and/or marketing purposes). For example, since animals may not be able to communicate symptoms of food sensitivity, identification of any food sensitivities present may improve quality of life (e.g., by reduction of symptoms) and/or decrease costs associated with animals (e.g., by decreased clinic visits). In addition, with the explosion of commercial offerings of premium, high-priced, "holistic" animal feed, animal feeding costs may potentially increase without health improvements when these types of feed are globally adopted. However, by providing routine testing and/or testing in response to suspected food sensitivities, a user may more adequately determine whether premium and/or less allergenic food would be beneficial to an animal or specific subset of animals. By identifying animals that may not benefit from premium and/or less allergenic foods, animal feeding costs may be decreased.

The veterinary diagnostic test may allow identification of one or more food sensitivities based on fecal samples. In some implementations, the veterinary diagnostic test (e.g., via testing on fecal samples) allows identification of a set of common food sensitivities (e.g., gluten) for a set of dogs.

Thus, feces of a dog may be obtained for testing rather than obtaining a blood sample. Obtaining blood samples from dogs may be invasive, difficult to obtain due to size of the dog, dangerous to the dog (e.g., taken from anesthetized dogs), and/or dangerous to the sample taker (e.g., dogs may be frightened and harm sample takers). Food sensitivities may be determined based on measuring Immunoglobin A (IgA) present in the stool sample, when stool samples are utilized to identify food sensitivities. Sampling blood for either IgA, IgG, or IgE antibodies may accompany and complement the fecal IgA testing, in some implementations.

The identification of food sensitivities may be utilized to diagnose symptoms, such as chronic gastrointestinal symptomatology (e.g., vomiting, diarrhea, constipation, foul/malodorous flatulence and/or stools, and/or abdominal bloating), behavioral and disobedience problems, or chronic immune/auto-immune sequelae; and/or specific disorders and diseases, including gluten-sensitive enteropathy, Crohn's Disease, and/or irritable bowel syndrome; reduce immunological sensitivities (e.g., by eliminating the identified food); and/or other appropriate purposes. In some implementations, the food sensitivities identified by testing fecal samples may be further tested and/or diagnoses of dogs may be further tested and/or confirmed using conventional methods, such as ELISA.

When a dog experiences sensitivity to a food, the food may act as an antigen in the dog and the dog may produce antibodies specific to the antigen (e.g., food). When a dog consumes an immunogenic food (e.g., that acts as an antigen in the body), the food specific IgA may bind to the antigen (e.g., the food) and cause an immunological reaction and/or symptom(s) (e.g., inflammation, diarrhea, nausea, etc.). The produced antibodies may include Immunoglobulin A (IgA). IgA is produced in mucosal linings and present in the gastrointestinal tract. Since IgA is produced in mucosal linings and present in the gastrointestinal tract, IgA, when produced by a dog, may be present in its feces. The IgA produced may be specific to a group of dogs (e.g., breeds of dogs, etc.). However, dogs can not communicate the presence of immunological reactions and/or most symptoms. Thus, a veterinary diagnostic test may facilitate identification of food sensitivities. The veterinary diagnostic test may be a part of routine testing and/or testing based on symptoms, hypotheses, and/or conjecture.

In various implementations, the veterinary diagnostic testing system includes a veterinary diagnostic test. The veterinary diagnostic test includes a test region.

The test region includes antibody binding agent. The antibody binding agent may include a compound with a receptor capable of selectively binding (e.g., the antibody binding agent may not bind with one or more other antibodies) with a predetermined antibody. For example, the antibody binding agent may include an antibody conjugate, such as an IgA conjugate (e.g., corn IgA conjugate, wheat IgA conjugate, etc). The antibody binding agent may be disposed on a substrate of the veterinary diagnostic test. The antibody binding agent may be applied to the test region prior to and/or before the fecal sample or portions thereof are disposed on the test region. The antibody binding agents may be bound (e.g., immobilized and/or coupled via methods similar to ELISA) on the test region, in some implementations. For example, antibody binding agents, such as antigens, may be bound on a substrate of the test region. In some implementations, the antibody binding agent may be coupled by passive adsorption to a test region of the substrate. For example, a solution of the antibody binding agent (e.g., in an alkaline buffer solution) may be contacted with substrate(s) and allowed to incubate. Then the solution may be removed or washed off such that antibody binding agents remain coupled to portion(s) of the substrate.

The antibody binding agent includes an agent adapted to couple with a predetermined antibody. The antibody binding agent may selectively bind with a predetermined antibody and inhibit binding with one or more other antibodies. For example, the antibody binding agent (e.g., IgA conjugate) may include a receptor adapted to couple with the predetermined antibody and adapted to not couple with other antibodies. The coupling between the antibody binding agent and the predetermined antibody may be chemical coupling and/or affinities between regions of the antibody binding agent and the predetermined antibody. For example, an antibody binding agent may include an antigen (e.g., corn IgA conjugate and/or other appropriate antigens) for a predetermined antibody (e.g., corn IgA, antigliadin IgA, antitissue transglutaminase IgA antibody; and/or other appropriate antibodies). When the antibody is in the presence of the antibody binding agent (e.g., antigen), the antibody and the antibody binding agent (e.g., antigen) may couple. For example, when a veterinary diagnostic test is adapted to identify corn sensitivities, the antibody binding agent may be adapted to selectively bind with corn IgA (e.g., IgA produced in response to an immunological food sensitivity reaction to corn) while being inhibited from coupling with other types of IgA, such as wheat IgA, egg IgA, corn IgE, etc. By utilizing antibody binding agents that selectively couple with predetermined antibodies (e.g., rather than coupling with predeterimined antibodies and other antibodies), false indications of food sensitivities may be reduced (e.g., when compared a test that utilizes nonselectively coupling antibody binding agents).

The veterinary diagnostic test may include a flag to provide notifications to a user. For example, the veterinary diagnostic test may include a flag that identifies predetermined couplings (e.g., between the antibody binding agent and the antibody, between a flag binding agent and the flag, etc.). The flag may be, for example, color markers, radioopaque markers, magnetic markers, fluorescent label (e.g., via a fluorophore), and/or any other appropriate flag (e.g., flags that provide audio, visual, and/or tactile indications). Utilizing flags may facilitate identification of predetermined couplings, such as between antibody binding agents and predetermined antibodies, and thus facilitate identification of food sensitivities. Utilizing flags may ease use by users, in some implementations. For example, a user may be able to view the veterinary diagnostic test and determine the results based on visualization of the flags or lack of flags. In some implementations, when the antibody binding agent and antibody bind, the flag may change colors (e.g., not visible to visible; white to red; red to blue; fluoresce; and/or combinations thereof). The user may then compare the changed color to an instruction set to determine if a food sensitivity exists based on the results (e.g., instruction set may include a color chart, color intensity chart, etc. that associates visual cues with results such as a specific set of food sensitivities).

In various implementations, the fecal sample may be processed prior to allowing a portion of the fecal sample (e.g., a testing portion) to contact the test region. In some implementations, processing the fecal sample may include coupling a flag to predetermined antibodies, if present, in the fecal sample. For example, a solution comprising the flags may be allowed to wash over at least a portion of the fecal sample. In some implementations, at least a portion of the fecal sample may be allowed to flow through a region of the veterinary diagnostic test that includes flags. If predetermined antibodies are present in the fecal sample, one or more of the flags may be coupled to the predetermined antibodies. In some implementations, processing the fecal sample may include adding a hydrating agent to the fecal sample and/or separating various portions of the fecal sample (e.g., centrifuging the fecal sample to allow removal of at least a portion of the solid material and/or supernatant fluid). For example, a fecal sample may be centrifuged at a rotational speed of approximately 13,500 rpm to approximately 20,000 rpm. The testing portion may be obtained from the supernatant liquid after centrifuging the fecal sample. In some implementations, the veterinary diagnostic test may be contacted to the supernatant liquid of the centrifuged fecal sample to allow a testing portion to flow to the test region of the veterinary diagnostic test.

In some implementations, the test region of the veterinary diagnostic test may include a control. The control may provide an indication that the veterinary diagnostic test is capable of identifying predetermined antibodies. For example, the control may include flag binding agents. The flag binding agents may be capable of selectively coupling with a predetermined flag. The flag binding agents may be capable of selectively coupling with flags present in the veterinary diagnostic test and may not be capable of binding with one or more other flags. In some implementations, the flag binding agent may be selected such that the flag binding agent is inhibited from coupling (e.g., the flag binding agent does not include a receptor capable of coupling) with the antibody that the veterinary diagnostic test seeks to identify, antibodies similar to the antibody sought to be identified, and/or other compounds. When a flag contacts the control of the test region, the flag may bind with the flag binding agent and provide an indication, such as a color change. For example, a veterinary diagnostic test may provide a first visual (e.g., a line or other shape from the flags) to show that fluid has contacted the control and a second visual (e.g., a line or other shape from the flags or no shape from the flags when antibodies have not bound to the antibody binding agent) to show the results of the veterinary diagnostic test such as positive reactivity for a set of food sensitivities (e.g., one or more food sensitivities).

In some implementations, the veterinary diagnostic test includes a substrate and one or more antibody binding agents. The substrate may include a plate (e.g., microtiter plate, nanoparticle plate, microfluidic array, sensor on chip, etc.), a container (e.g., cup and/or bowl), a test strip, a stick, and/or other appropriate testing substrate. In some implementations, the fecal sample or portions thereof may be collected directly in the substrate, may be absorbed by the substrate, may flow through the substrate (e.g., via capillary action), and/or be transferred to the substrate of the veterinary diagnostic test.

In some implementations, a veterinary diagnostic testing system may include a plate based testing system. The veterinary diagnostic testing system includes a veterinary diagnostic test that includes a substrate. The substrate may include a plate, such as a microtiter plate, sensor on chip, and/or microfluidic array. The substrate may be used with enzyme linked immunoassay (ELISA) and/or other assay detection methods. For example, the substrate may allow coupling of antibodies in the fecal sample to the substrate. In some implementations, the antibodies may be coupled to discrete regions of the substrate. One or more washes may be performed, as in ELISA methods to remove other compounds from the fecal sample. A solution including flags may be allowed to contact the coupled antibodies from the fecal sample. The flags may be selectively coupling flags that couple with some antibodies and may be restricted from coupling with other antibodies. The flags may couple with one or more predetermined antibodies coupled to the substrate. The ELISA method may be further continued to produce an indication of the presence of predetermined antibodies, an amount (relative, absolute) of predetermined antibodies in the sample, etc. The indication may include color change(s), color intensity, number of localizations of color, visualization of fluorophores, and/or any other appropriate indication (e.g., visual, auditory, and/or tactile). An identification of one or more possible food sensitivities may be based at least partially identification of the predetermined antibodies in the sample.

In some implementations, the plate based veterinary diagnostic testing system may be used in clinical and/or laboratory setting.

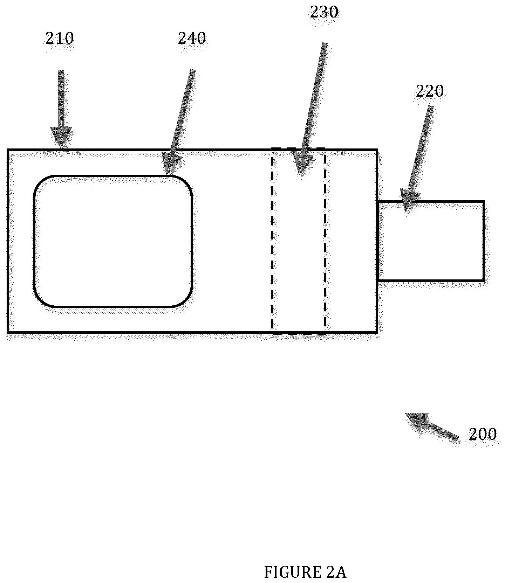

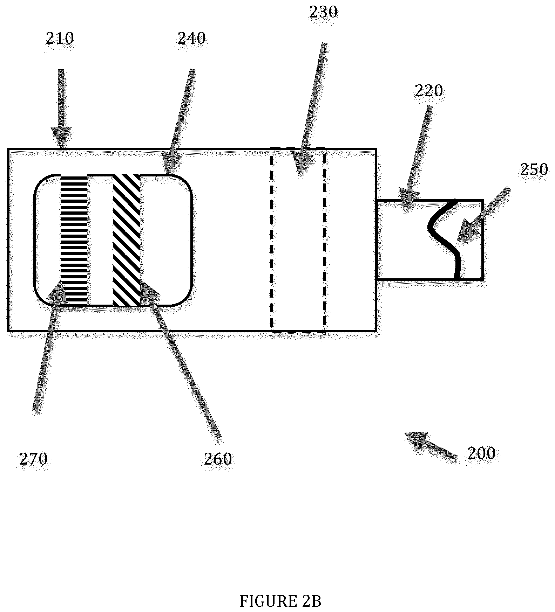

In some implementations, the veterinary diagnostic testing system may include a veterinary diagnostic test stick. The veterinary diagnostic test stick may be any appropriate shape, such as a rectangular prism, ellipsoid, conical, cup-shape, and/or any other appropriate regular or irregular shape. Figure 2A illustrates an implementation of an example of stick type of a veterinary diagnostic test 200. Figure 2B illustrates the veterinary diagnostic test 200 during use. The veterinary diagnostic test 200 may allow rapid, easy to read results. Using a stick based veterinary diagnostic test may be easier to use for users, since the stick may not include steps such as washing plates with reagents, applying flags, and/or specialized visualization of flags (e.g., via microscope, via radioimaging, and/or via other visualization methods).

At least a portion of the veterinary diagnostic test stick 200 may be disposed in a housing 210. The housing 210 may allow a user to hold the veterinary diagnostic test stick 200 without touching one or more regions of the veterinary diagnostic test stick (e.g., the absorbent region 220). The stick based veterinary diagnostic test 200 may include an absorbent region 220, a flag region 230, and a test region 240. The regions of the veterinary diagnostic test 200 may be on a continuous substrate, such as an absorbent pad, woven fibers (e.g., synthetic and/or natural fibers), and/or paper, in some implementations. In some implementations, the regions of the veterinary diagnostic test may not be continuous but may allow fluids (e.g., fecal sample, flags, etc.) to flow from a first region to another region.

The absorbent region of the veterinary diagnostic test stick may draw fluid (e.g., via capillary action and/or absorption) into the veterinary diagnostic test stick. For example, the absorbent region may draw fluid from a fecal sample at least partially into the veterinary diagnostic test stick. In some implementations, water may be added to the fecal sample to facilitate drawing at least a portion of the fecal sample into the veterinary diagnostic test stick. In some implementations, the fecal sample may include enough water to allow at least a portion of the fecal sample (e.g., water and/or antibodies in the fecal sample) into the veterinary diagnostic test stick. In some implementations, the fecal sample may be separated (e.g., via a centrifuge) and the veterinary diagnostic stick may be inserted into the supernatant liquid of the separated fecal sample. The absorbent region may draw fluids into at least a portion of the veterinary diagnostic test stick by capillary action, absorption, pressure differentials, pumping (e.g., via pipette and bulb), and/or any other appropriate fluid transportation mechanism.

The absorbent region of the veterinary diagnostic test stick may be placed in contact with the fecal sample (e.g., by pushing an absorbent pad of the absorbant region into the fecal sample, by placing the absorbent region in the supernatant water collected from a centrifuged fecal sample, etc.). As illustrated in Figure 2B, the absorbent region 220 may transport one or more fluids 250 from the fecal sample through the absorbent region and to the flag region 230 of the veterinary diagnostic test 200.

In the flag region, the testing portion of the fecal sample (e.g., a portion of the fecal sample) may flow at least partially through the flag region. In the flag region, flag may selectively bind to predetermined antibodies, if these predetermined antibodies are present in the testing portion of the fecal sample. In some implementations, flags in the flag region of the veterinary diagnostic test may be released into the testing portion of the fecal sample as it passes through the flag region and to the test region. The flags may bind to the antibody binding agents and/or antibodies. In some implementations, the veterinary diagnostic test stick may not include a flag region. For example, the fecal sample may be contacted with flags (e.g., wash and/or apply a solution including flags to the fecal sample) prior to placing the veterinary diagnostic test stick in contact with the fecal sample.

The veterinary diagnostic test stick may include a test region. The test region may include antibody binding agents. The antibody binding agents may include any appropriate compound (e.g., enzyme, reagent, antigen) that is capable of selectively binding with a predetermined antibody. In some implementations, the antibody binding agent for an IgA antibody may include the associated antigen. For example, the antibody binding agent for the IgA antibody associated with corn sensitivity may include corn antigen (e.g., corn as the antigen). If IgA antibody associated with corn is present in a fecal sample, it will bind with corn antigen. The corn antigen may not bind with other IgA present in the fecal sample, such as IgA associated with dairy. Thus, a sensitivity to corn may be identified through the coupling of the antibody with an antibody binding agent.

In the test region, the flag may be visible or otherwise apparent (e.g., tactile signal) when the antibody, the antibody binding agent, and the flag are coupled. Thus, the presence of a predetermined antibody may be identified based on the visualization or other identification of the flag. As illustrated in Figure 2B, when the flag is coupled to the control (e.g., including flag binding agents), a visible signal 260 may be produced in the test region 240. When the antibody is coupled to the antibody binding agent, the flag may produce, for example, a visible signal 270 in the test region 240. In some implementations, the test region may include an immunoassay strip.

In some implementations, the flag region and the test region of the veterinary diagnostic test stick may be combined. In some implementations, the flags may be coupled to the control and/or antibody binding agents. For example, flags may be coupled to the antibody binding agents and/or with the control region of the veterinary diagnostic test stick. In some implementations, the flags may be disposed in the test region and as fluid (e.g., from the fecal sample) travels at least partially through the test region, the fluid may contact flag(s) and/or antibody binding agents. In some implementations, the control may indicate the presence of fluid in the control region rather than the presence of a predetermined flag to provide an indication that the veterinary diagnostic test is capable of operating to identify food sensitivities.

The veterinary diagnostic test allows food sensitivities to be identified based on signals provided by the veterinary diagnostic test. For example, a user may select a set of food sensitivities for which to test. Since the antibodies for different foods may be different, a test is associated with a set of food sensitivities based on which antibody binding agents are present in the test region. The user may then select the appropriate veterinary diagnostic test based on the type of dog to be tested. For example, the antibody binding agents on the veterinary diagnostic test may be different to allow the coupling between the antibody and antibody binding agent. The user (e.g., while holding the housing of the veterinary diagnostic test) may allow the absorbing region of the veterinary diagnostic test to contact a fecal sample (e.g., feces or portions thereof) from the dog. The fecal sample may include fluids (e.g., water, urea, and/or mucus) and the fluids may include antibodies, if present in the fecal sample. The fluid may be drawn (e.g., via capillary action, absorption, pressure differential, and/or or otherwise drawn) between the various regions of the veterinary diagnostic test. At least a portion of the fluid from the fecal sample may be drawn into the absorbent region of the veterinary diagnostic test (e.g., via capillary action, absorption, drawn to materials in the absorbent region). At least a portion of the fluid in the absorbent region may be allowed to flow from the absorbent region to the flag region. In the flag region, if antibodies, associated with the food sensitivities for which the test is selected, are present in the fluid from the fecal sample, the antibodies may couple with flag(s). For example, the flag(s) may be embedded in the material of the flag region and as the fluid from the fecal sample passes through the flag region, the flags may be released and/or selectively bind with one or more specific antibodies, if present. The specific antibodies may be the antibodies associated with the food sensitivities for which the veterinary diagnostic test is selected. At least a portion of the fluid, possibly including the specific antibodies bound to flags, may then pass to the test region. In the test region, antibody binding agents may be present (e.g., coupled to the test region, washed over the test region). At least a portion of the fluid from the flag region may pass proximate the antibody binding agents. If specific antibodies are present in the fluid, the antibody binding agents and the specific antibodies may couple. When the antibody binding agent and the specific antibody couple, the flag may provide an indication (e.g., visible color) of the presence of the coupled antibody. In some implementations, the veterinary diagnostic test may include a control. The control may, for example, include flag binding agents. As the fluid from the fecal sample passes through the test region, the fluid may pass proximate the control. The flag binding agents may couple with flags (e.g., coupled flags and/or uncoupled flags) in the fluid. In some implementations, the antibody binding agents and/or the control may be coupled to the test region in a specific pattern to facilitate identification (e.g., lines, circles, etc.). In some implementations, an intensity of the indication provided by the flags (e.g., intensity of color) may indicate a level (e.g., an approximate relative amount, such as a ratio, and/or an approximate absolute amount) of antibodies present in the fecal sample. Thus, based on the indication provided by the flags in the test region, a user may be able to identify if the test was executed appropriately (e.g., when the control provides an indication), whether specific antibodies are present, and/or a level of antibodies.

The user may utilize the results of the veterinary diagnostic test to provide an initial and/or dispositive indication of a food sensitivity associated with the set of antibodies (e.g., one or more antibodies) for which the test was selected. In some implementations, the user may follow up the results with additional home testing, clinical testing, and/or laboratory testing of fecal samples, blood samples, and/or other testing (e.g., examination of intestines). After food sensitivity is identified using the veterinary diagnostic test, disorders and/or diseases associated with the food sensitivity may be identified based at least partially on the one or more food sensitivities identified using the veterinary diagnostic test. For example, if gluten sensitivity is identified by the veterinary diagnostic test, Crohn's disease may be identified in the dog based on the results of the veterinary diagnostic test and/or other medical information about the dog (e.g., weight loss, no cancer detected).

In some implementations, the veterinary diagnostic testing system may include a home kit. The home kit may include a fecal sample container, veterinary diagnostic test, and/or instructions. In some implementations, the veterinary diagnostic testing system may include one or more veterinary diagnostic test sticks. The veterinary diagnostic testing system may include instructions (e.g., wait times, whether to add reagents or hydrating agents, how to read test region and/or control, etc.).

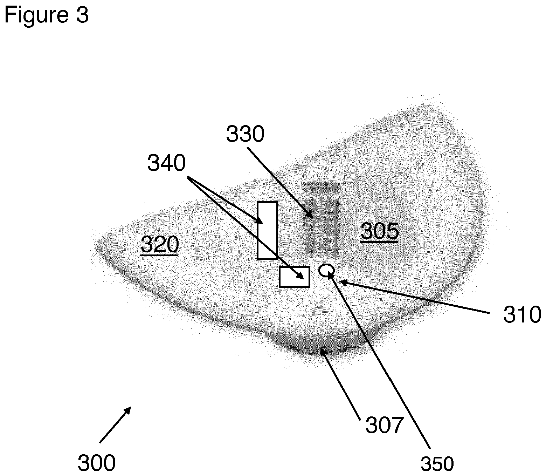

In some implementations, the fecal collection tool may include an integrated food sensitivity test. For example, the fecal collection tool may include a shape that facilitates collection of feces from a dog (e.g., a bucket like shape, hat shaped, a shovel like shape, etc.). Figure 3 illustrates an implementation of an example fecal collection tool 300 with an integrated food sensitivity test. The fecal collection tool may include an inner surface and an outer surface. As illustrated, the fecal collection tool 300 includes a collection portion 310 and a rim 320. The collection portion 310 may be shaped and sized to hold a fecal sample. The rim 320 may facilitate fecal collection and/or holding the fecal collection tool. As illustrated, the rim 320 may extend (e.g., radially) from the collection portion 310. In some implementations, the rim may extend in one or more directions to allow attachment an object to facilitate fecal sample collection; to facilitate holding the fecal collection tool, and/or for any other appropriate purpose. In some implementations, the rim may be collapsible (e.g., the rim may include radial flanges that can be collapsed, the rim may include flanges that collapse in, the rim may include flanges that fold inwards to form a cover, etc.). As illustrated the fecal collection portion 310 may have walls and a bottom surface. The collection portion 310 may include measuring tools 330, such as markings to indicate volume or any other appropriate measuring tool. The measuring tools may be disposed on an inner surface of the fecal collection tool 300. The veterinary diagnostic test 340 may be integrated with the collection portion 310. As illustrated, the veterinary diagnostic test 340 may be positioned on a wall of the collection portion and/or on a bottom of a collection portion. In some implementations, multiple veterinary diagnostic tests may be provided on a fecal collection tool. The veterinary diagnostic test(s) 340 may be positioned on an inner surface 305 of the fecal collection tool, as illustrated. An opening 350 may be disposed in the fecal collection tool (e.g., to allow fluids from the fecal sample, flag solution, hydrating fluids, and/or the fecal sample or portions thereof to drain from the fecal collection tool). As illustrated, the opening 350 may be disposed proximate a bottom of the fecal collection tool. In some implementations, a removable stop (e.g., a stop that can be removed and/or reinserted) (not shown) may be disposed in the opening 350 to inhibit fluids and/or solids from flowing through the fecal collection tool.

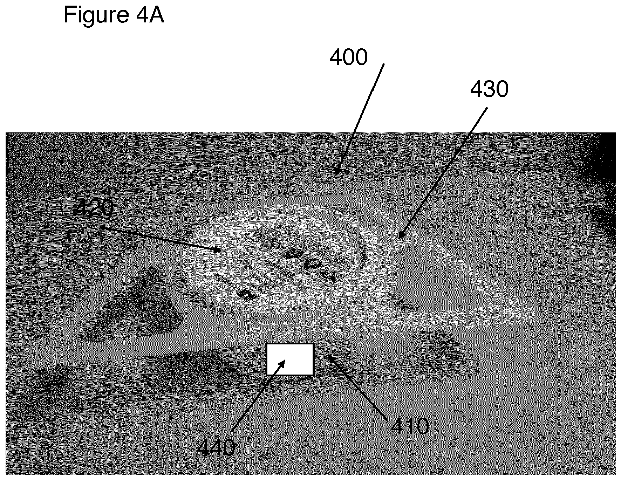

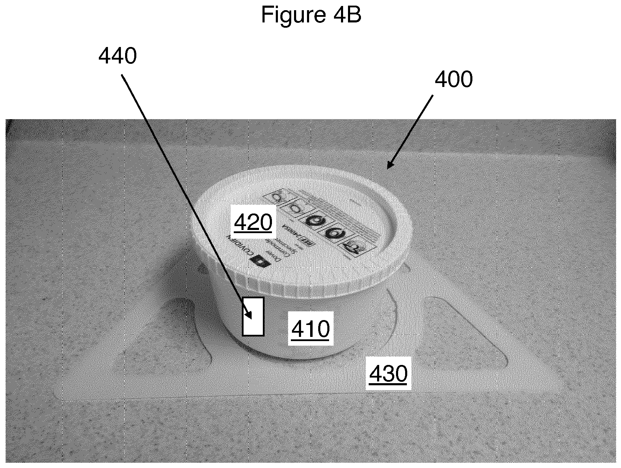

Figure 4A illustrates an implementation of an example fecal collection tool 400 and Figure 4B illustrates the fecal collection tool 400 in which the collar has been removed. As illustrated, fecal collection tool 400 includes a collection portion 410, cover 420, and a collar 430. The cover 420 may allow the collection portion to be closed (e.g., for transport, for disposal, etc.). The collar 430 may be removable. As illustrated, the collar may include an opening in which the collection portion 410 can be disposed (e.g., without falling through the opening). The collar 430 may include a rim to facilitate positioning of the fecal collection tool. In some implementations, the collar 430 may include an opening to receive the collecting portion 410 and a handle to allow a user to hold the fecal collection tool without contacting the fecal sample. The collar may be attached during fecal sample collection and removed, for example, to view test results, send the fecal sample to a laboratory for further testing, etc. The fecal collection tool may include a veterinary diagnostic test (not shown) on an inner surface of the collection portion. During use, at least a portion of the fecal sample may contact or be allowed to contact (e.g.,a user may move a portion of the fecal sample to contact) the veterinary diagnostic test. As illustrated, the testing portion 440 and/or results (not shown) of the veterinary diagnostic test may be visible from an outer surface of the fecal collection tool. For example, the collection portion may be formed of a transparent and/or translucent material that allows viewing of the food sensitivity test from an outer surface. In some implementations, the veterinary diagnostic test may be disposed in an opening of the fecal collection tool and the veterinary diagnostic test may be viewable from the outer surface. In some implementations, the absorbent region of the veterinary diagnostic test may be disposed on an inner surface of the fecal collection tool, to allow contact between the absorbent region and the fecal sample or portions thereof. The testing portion or portions thereof may be disposed on an outer surface of the fecal collection tool to allow viewing of the testing portion and/or results.

The fecal collection tool may include pan with an opening to allow liquids (e.g., urine) and/or solids to drain from the pan, while the pan is capable of holding fecal samples. The opening may allow the fecal sample to be drained from the fecal collection tool after testing (e.g., for disposal and/or into another container for storage and/or further testing). The opening may be disposed in a bottom potion and/or side portion(s) of the fecal collection tool. The fecal collection tool may include one or more opening(s) that may have any appropriate position, size (e.g., large enough to allow a fluid to drain and/or not large enough to allow the fecal sample to fall through the opening if a stop is not utilized) and/or shape (e.g., circular, rectangular, etc.). For example, the fecal collection tool may include a plurality of slits that act as openings to allow at least a portion of the fluid to drain from the fecal collection tool. In some implementations, the openings may include an opening with a stop (e.g., removable). For example, the stop may not block the opening during sample collection (e.g., such that urine may pass); the stop may then be allowed to block the opening to allow testing and/or fecal sample processing. In some implementations, the stop may be allowed to block the opening during fecal sample collection and the stop may be removed at a later point (e.g., to drain at least a portion of urine in the fecal collection tool, to drain at least a portion of a flag solution applied to the fecal sample in the fecal collection tool, to drain at least a portion of hydrating solution applied to the fecal sample, and/or to remove the fecal sample from the fecal collection tool).

The fecal collection tool may include a handle to allow a user to hold the fecal collection tool in place while not contacting feces from the dog. The fecal collection tool may include a test region disposed in an area of the pan in which fecal samples may be collected. For example, one or more walls of the pan of a fecal collection tool may be slanted to promote contact of the fecal sample with the test region of the food sensitivity test. The fecal sample may be allowed to remain in contact with a fecal sample collected in the fecal collection tool for a first period of time. As the fecal sample is in contact with the test region, antibodies, if present in the fecal sample, may contact with flags and/or antibody test regions. In some implementations, water or another hydrating agent may be provided in the pan to at least partially hydrate the fecal sample and/or facilitate fluids from the fecal sample flowing proximate and/or in the test region. After the first period of time the fecal sample may be moved from the area proximate the test region, removed from the pan and/or the pan may be rinsed (e.g., with water). The user may then be able to view the test region and/or indications provided by flags. Food sensitivities may be identified from the indications, as previously described.

In some implementations, the specimen container and the food sensitivity test may be integrated. For example, a specimen container may include a test region. For example, the test region may be disposed on a wall or bottom of the container. A portion of a fecal sample may be transferred (e.g., from feces on the ground) to the specimen container. The fecal sample may contact the test region and/or water may be added to allow the fecal sample to hydrate and/or contact the test region. As the fecal sample is in contact with the test region, antibodies, if present in the fecal sample, may contact with flags and/or antibody test regions to provide indications. The indications may indicate the presence or absence of food sensitivities. In some implementations, after the first period of time the fecal sample may be moved from the area proximate the test region, removed from the pan and/or the pan may be rinsed (e.g., with water). The user may then be able to view the test region and/or indications provided by flags. Food sensitivities may be identified from the indications, as previously described.

In some implementations, the veterinary diagnostic test may be used to determine whether a dog has a set of food sensitivities. A set of food sensitivities may include one or more food sensitivities. For example, a test may be utilized to determine if a dog has food sensitivities to a set of grains. A test may be utilized to determine if a dog has food sensitivities to one or more food substances (e.g., grain, dairy, and/or eggs, common allergens), in some implementations. Additional testing (e.g., additional veterinary diagnostic tests and/or other tests) may be performed if the veterinary diagnostic test indicates sensitivity to one or more of the food sensitivities associated with the veterinary diagnostic test.

In some implementations, a veterinary diagnostic test that is associated with a plurality of food sensitivities may or may not distinguish between the indications provided for each of the food sensitivities. For example, a veterinary diagnostic test may be associated with corn sensitivity, wheat sensitivity, and supplement sensitivity. The flag region may include one or more flags that selectively bind with one or more of the antibodies associated with each of these sensitivities. The flag coupled to a first antibody, such as corn antibody (e.g., the antibody associated with a corn sensitivity), may provide the same indication (e.g., blue and/or fluoresce) as the flag or a different flag in the flag region coupled with one or more of the other antibodies associated with the test (e.g., wheat antibody and/or supplement A antibody). Thus, the indication provided by the flag in the test region may identify one or more food sensitivity (e.g., by the indication provided by the flags coupling with antibodies and antibody binding agents) and may not distinguish between the different food sensitivities.

In some implementations, when a veterinary diagnostic test is associated with testing a plurality of food sensitivities, the veterinary diagnostic test may allow distinguishing between the food sensitivities identified. For example, each food sensitivity may be associated with a flag that provides a specific indication (e.g., a specific color). In some implementations, the food sensitivity may be associated with a particular part of the test region. For example, the antibody binding agents may be coupled to the test region in lines, and so when an indication is provided along a line of the test region, the food sensitivity type may be determined based on the location of the line.

In some implementations, a user (e.g., veterinarian, a laboratory worker, a dog owner) may select a veterinary diagnostic test based on the type of dog to be tested. A veterinary diagnostic test may allow testing of a set (e.g., one or more) of predetermined group of dogs (e.g., same species of dogs, a subspecies of dog, etc.) for a predetermined set of food sensitivities (e.g., since the antibodies produced by various dogs may be different or the same). The veterinary diagnostic test may include antigen binding agents and flags that are capable of coupling with the antibodies produced by the set of dogs associated with the veterinary diagnostic test.

In some implementations, a fecal sample may be obtained from a dog via a provided specimen container. In some implementations, a user may collect a fecal sample. For example, the veterinary diagnostic test and/or kit may include a specimen container (e.g., jar, tray). The user may use a collection tool, such as a tongue blade, to transfer a portion of feces collected from a dog to the specimen container.

In some implementations, the fecal sample may be further processed prior to testing the fecal sample. The fecal sample may be concentrated. For example, the fecal sample may be placed in a centrifuge (e.g., to be rotated at speeds of approximately 13,500 rpm to approximately 20,000 rpm) and the supernatant fluid may be tested. In some implementations, the fecal sample may be freeze dried (e.g., lyophilized). Water may be added to the freeze dried fecal sample prior to testing the fecal sample. In some implementations, freeze drying the fecal sample may facilitate testing of different types of fecal matter (e.g., watery fecal matter).

In some implementations, the fecal sample may not be placed into a specimen container prior to testing the fecal sample with the veterinary diagnostic test. For example, a user may insert a stick based veterinary diagnostic test directly into feces from a dog. In some implementations, water may be added to the feces and/or the feces may be mixed with water prior to inserting the veterinary diagnostic test into the feces.

In some implementations, a set of veterinary diagnostic tests may be provided in a kit. The kit may include a set of veterinary diagnostic tests (e.g., one or more veterinary diagnostic tests). The set of veterinary diagnostic tests may include food specific veterinary diagnostic test for one or more sets of dogs. For example, one or more gluten tolerance veterinary diagnostic tests (e.g., a veterinary diagnostic test to identify gluten sensitivities) may be provided and each of the gluten tolerance veterinary diagnostic tests may be for different types of dogs. In some implementations, the set of veterinary diagnostic tests may include veterinary diagnostic tests for one or more sets of food sensitivities for a predetermined group of dogs.

In some implementations, the veterinary diagnostic test may allow testing of a plurality of antibodies associated with a single food (e.g., corn, dairy, eggs, a specific supplement). Since a food, food A, may include more than the part that may trigger immunological reactions, a dog may produce more than one type of antibody in response to contact (e.g., touching, consuming, etc.) with food A. For example, a species of fish may have several different types of proteins within the fish. A dog that contacts the fish, by for example eating the fish, may produce more than one type of antibody in response, if the dog has an immunological reaction to the fish. In some dogs, the dog may have a reaction to a first protein in the fish but not have a reaction to a second protein in the fish. An immunological reaction to each of these proteins may produce different antibodies. Thus, if an immunological test includes an antibody binding agent that selectively couples to the antibody produced in response to the first protein and not the antibody produced in response to the second protein, the immunological test may produce a false negative (e.g., the dog has a food sensitivity but it is not identified by the test). Thus, a veterinary diagnostic test may include antibody binding agents for more than one protein associated with a food to decrease the rate of false negatives (e.g., when compared with a test that only includes a single type of antibody binding agent). The flags may produce indications for each of the antibody - antibody binding agent bindings that are similar or different based on the type of protein associated with the antibody. For example, a home kit may include several types of proteins but not distinguish between sensitivities to the several proteins (e.g., since a user may not care which protein a dog has a sensitivity but has concerns about a particular food). In some implementations, a laboratory kit may include a veterinary diagnostic test that distinguishes between indications associated with different proteins (e.g., to provide a more detailed analysis, to identify other sources of the protein that may cause immunological reactions, etc.).

In some implementations, the veterinary diagnostic testing system may include utilizing successively specific veterinary diagnostic tests to reduce the number of tests to perform, to facilitate usability by the user, to reduce costs, to provide specificity requested from a user, etc. For example, one or more first veterinary diagnostic test may be used to identify whether a dog has food sensitivities to a first set of foods. If the results are negative (e.g., no food sensitivity detected), then rather than having to purchase and perform a test for each of the foods individually a single test may provide results. If a result is positive (e.g., a food sensitivity detected), then additional testing may be performed using second veterinary diagnostic tests. For example, the second veterinary diagnostic tests may identify a smaller second set of food sensitivities than the first set of foods. The second set may be associated with one or more foods. Additional tests, such as third veterinary diagnostic tests may be performed based on the results of the second veterinary diagnostic tests.

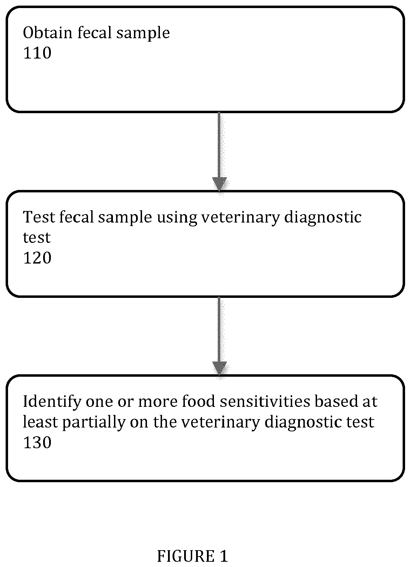

In various implementations, a veterinary diagnostic test may allow identification of a set of food sensitivities in one or more dogs. Figure 1 illustrates an implementation of an example of a process for identifying food sensitivities. A fecal sample may be obtained (operation 110). The fecal sample may be tested using the veterinary diagnostic test (operation 120). During testing of fecal samples with the veterinary diagnostic test, the veterinary diagnostic test may be contacted with a fecal sample. For example, supernatant liquid from a fecal sample may be deposited on the veterinary diagnostic test. In some implementations, the veterinary diagnostic test may be dipped into the fecal sample. The fecal sample may be processed to bind a flag (e.g., color, fluorescence, magnetic flag, and/or other detectable indicators) to a predetermined antibody, if present in the fecal sample. The flag may be activated (e.g., the color may change, the flag may fluoresce, etc.) when the flag is coupled to an antibody binding agent, in some implementations. In some implementations, fluids in at least a portion of the fecal sample may flow through (e.g., through a chamber, through channel(s), etc.) a flag region such that predetermined antibodies, if present in the fecal sample, may contact and couple with the flag. Thus, when the veterinary diagnostic test is contacted with the fecal sample, if antibodies are present in the fecal sample, the antibodies will couple with the antibody binding agents on the substrate of the test and the flag may be activated.

One or more food sensitivities may be identified based at least partially on the veterinary diagnostic test (operation 130). For example, the fecal sample obtained from a dog may include food specific IgA, in levels detectable by the veterinary diagnostic test. Activation of the flag(s) in the test region may provide indicia to facilitate identification of the predetermined antibody in the fecal sample, and thus food sensitivity related the predetermined antibody.

In some implementations, the veterinary diagnostic test may include a test strip. The test strip may include antibody binding agent that when coupled to a predetermined antibody produces an indicia (e.g., color change, fluorescence, etc.).

In some implementations, the fecal sample may be processed and/or unprocessed prior to allowing the veterinary diagnostic test to identify food sensitivities. For example, the fecal sample may be obtained and an undiluted and/or unconcentrated fecal sample may be utilized with the veterinary diagnostic test. In some implementations, a veterinary diagnostic test may be dipped into an undiluted and unconcentrated fecal sample.

In some implementations, the fecal sample may be concentrated prior to allowing the veterinary diagnostic test to identify food sensitivities. A fecal sample may be obtained and concentrated. For example, the fecal sample may be centrifuged (e.g., at approximately 13,500 rpm - approximately 20,000 rpm or any appropriate velocity). The supernatant liquid from the centrifuged fecal sample may be collected, in some implementations. The veterinary diagnostic test may be performed on the supernatant liquid. For example, the veterinary diagnostic test may be dipped into the supernatant liquid to identify food sensitivities. In some implementations, at least a portion of the supernatant liquid may be deposited on the substrate and/or the test region of the veterinary diagnostic test.

In some implementations, the fecal sample may be overly watery (e.g., due to diarrhea). The fecal sample may be concentrated to facilitate identification of food sensitivities. For example, the fecal sample may be freeze dried. The solid freeze dried fecal sample obtained may then be reconstituted with a hydrating agent (e.g., water) to an appropriate moisture content (e.g., to allow appropriate testing using the veterinary diagnostic test). For example, the percentage of dry to wet may be approximately 25 percent to approximately 75 percent and/or any other appropriate ratio. In some implementations, the freeze dried fecal sample may be reconstituted to a similar moisture content of non-diarrhea stool (e.g., less than the moisture content of the original fecal sample).

In some implementations, the fecal sample may be overly dry (e.g., due to constipation or the species of dog). The fecal sample may be hydrated to allow moisture to transport the antibodies, if present in the fecal sample, into the veterinary diagnostic test. For example, water or another hydrating agent may be added to the fecal sample. The hydrating agent may be added to the fecal sample to obtain a moisture content in a predetermined range (e.g., approximately 25 percent to approximately 75 percent, a moisture content associated with nonconstipated stool, a moisture content that allows transportation of antibodies present in the fecal sample through the absorbent region of the veterinary diagnostic test and/or any other appropriate moisture content). In some implementations, the fecal collection tool may be used to mix the fecal sample and the water. The hydrated fecal sample may then be utilized with the veterinary diagnostic test. For example, the veterinary diagnostic test may be dipped into the hydrated fecal sample.

In some implementations, one or more veterinary diagnostic tests may be performed on the same fecal sample (e.g., the same testing portion and/or another testing portion from the same fecal sample). For example, a veterinary diagnostic test may be associated with a first set of foods (e.g., common allergens, gluten panel, grain panel, additives panel, and/or any other appropriate set that can be performed together). The first veterinary diagnostic test may produce a first result (e.g., food sensitivity to one or more of the first set of food sensitivities). Subsequent veterinary diagnostic test(s) may be performed based on the first results from the first veterinary diagnostic test. For example, if a first veterinary diagnostic test does not distinguish between the food sensitivity in the first set (e.g., a positive or negative reactivity is produced for the overall set), and the first result is positive reactivity then one or more subsequent veterinary diagnostic tests may be performed to specify the identify of the food sensitivity (e.g., the subsequent test may include a portion of the food sensitivities in the first set of food sensitivities associated with the first test). In some implementations, if a first veterinary diagnostic test is negative, then subsequent veterinary diagnostic test(s) may be performed with one or more second sets of food sensitivities (e.g., including at least one different food sensitivity from the first set of food sensitivities). In some implementations, by testing for multiple food sensitivities with one veterinary diagnostic test, costs may be reduced and results may be obtained more quickly (e.g., when compared with running a screening of a plurality of food sensitivities).

In some implementations, a user may purchase a kit of veterinary diagnostic test(s) and select one or more to perform on a fecal sample. In some implementations, a user may purchase a kit with a fecal collection tool integrated with one or more veterinary diagnostic test(s) and one or more separate veterinary diagnostic test(s). The user may determine which, if any separate veterinary diagnostic test(s) to perform based on the results from the veterinary diagnostic test(s) integrated with the fecal collection tool. For example, a user use separate veterinary diagnostic test(s) associated with different sets of food sensitivities and/or subsets of the food sensitivities tested for using the integrated veterinary diagnostic test(s). In some implementations, the separate veterinary diagnostic test(s) may be utilized for confirmatory testing.

In some implementations, the veterinary diagnostic test may be utilized to determine efficacy of diet changes. For example, a set of food sensitivities for a dog may be identified (e.g., based on the veterinary diagnostic test result) and the dog's diet may be altered to reduce and/or eliminate one or more of the foods identified. Subsequent veterinary diagnostic test(s) may be performed to determine whether overall sensitivity has been reduced. In some implementations, one or more foods may be added back into the diet based on the subsequent testing. For example, if a dog has a "leaky gut" based on inflammation, the dog may have increased overall sensitivity and an initial test may show positive reactivity (e.g., positive for food sensitivity) for multiple foods. After elimination of one or more of these foods (e.g., in the set associated with positive food sensitivity), the dog's overall inflammation and/or overall sensitivity may be decreased. The decrease in inflammation and/or sensitivity (e.g., when compared with the initial test), may identify the foods for which the dog has food sensitivity and which foods that were previously indentified as in the set of foods to which the dog is sensitive were misidentified based on the overall inflammation of the dog. For example, an initial test may identify that A, who has a "leaky gut," has food sensitivity to wheat, cow's milk, and soy. After elimination of wheat, cow's milk, and soy from the diet of A for a first predetermined period, subsequent veterinary diagnostic test(s) may be performed on fecal samples from A. The subsequent test results may indicate that wheat sensitivity is present but sensitivity to cow's milk and soy is not present. The reduction in the sensitivity to cow's milk and soy may be based on a reduction in overall inflammation and/or sensitivity, and the diet of A may be adjusted to all reintroduction of one or more of these items.

### EXAMPLES

### EXAMPLE 1

Fresh stool was collected from 8 canines. Each stool sample was homogenized (e.g., stirred and/or agitated). The homogenized stool sample was centrifuged such that a liquid (e.g., supernatant liquid) was separated from solids. A portion of the supernatant liquid was obtained as the testing portion of each of the fecal samples. The testing portions were analyzed using veterinary diagnostic tests.

Each veterinary diagnostic test included a set of Canine IgA Conjugate coupled to a substrate. Each Canine IgA Conjugate (e.g., a set of antibody binding agent) was associated with a food in a set of foods. The testing portions of each of the fecal samples was contacted with the veterinary diagnostic test and a determination was made whether Canine IgA associated with a food sensitivity coupled to a Canine IgA Conjugate on the veterinary diagnostic test (e.g., visually determined, for example, based on flags of the veterinary diagnostic test). A canine was determined to have sensitivity to a food in the set of 15 foods if a determination is made that the Canine IgA associated with the food was present in the testing portion (e.g., the presence was determined based on if the Canine IgA bound with the Canine IgA Conjugate on the veterinary diagnostic test). Clinical outcomes of the animals were monitored following the outcome of the veterinary diagnostic test to confirm and/or monitor the affect of the food sensitivity (e.g., improvement of health based on elimination of an identified food for which sensitivity was identified).

Table A includes the results from the testing on the 8 canines. Values less than 10 indicate a negative reactivity (e.g., more than a predetermined amount of Canine IgA in the testing portion was not found to be coupled to the Canine IgA Conjugate on the veterinary diagnostic test) and values greater than 10 indicate a positive reactivity (e.g., food sensitivity is present based on the analysis of the testing portion). As illustrated, most canines appear to have a positive reactivity to other grains that can include gluten, such as corn and oats, and less reactivity to grains with little or no gluten, such as rice. As illustrated in Table A, canines may have food sensitivity in foods canines commonly eat but may not have food sensitivity to foods canines do not commonly eat, such as nuts. This is unexpected, since in humans, food sensitivities may be present to foods not commonly in a human's diet.

**Table A: Food sensitivity results for canines based on veterinary test**

| | **Canine A** | **Canine B** | **Canine C** | **Canine D** | **Canine E** | **Canine F** | **Canine G** | **Canine H** |
|---|---|---|---|---|---|---|---|---|
| **Corn** | **12** | **12** | **15** | **13** | **11** | **11** | 7 | **15** |
| **Oats** | **12** | **13** | **16** | 9 | **14** | **10** | 9 | **10** |
| **Rice** | 6 | **10** | **10** | 6 | 5 | 8 | 5 | 6 |
| **Beef** | 7 | **10** | 8 | 5 | 8 | 6 | 5 | 5 |
| **Chicken** | 5 | **13** | 9 | 6 | 7 | 9 | 4 | 6 |
| **Pork** | 8 | 9 | **10** | 6 | 5 | 7 | 4 | 6 |
| **Tuna** | 9 | **12** | **15** | **11** | 9 | **16** | 9 | 7 |
| **Almonds** | 7 | **15** | 9 | 5 | 5 | 8 | 4 | 5 |
| **Walnuts** | 7 | **21** | 9 | 3 | 2 | 6 | 2 | 3 |
| **Cashews** | 7 | 6 | 9 | 4 | 4 | 8 | 3 | 4 |
| **White Potato** | 7 | **11** | **11** | 7 | 9 | **10** | 6 | 9 |

In some implementations, the veterinary diagnostic test on fecal sample may accompany clinical observations and/or blood tests. Clinical observations regarding Canine B indicated that Canine B experienced "leaky gut," often characterized by enhanced intestinal permeability usually due to an inflammatory state that may cause pan-sensitivity. As shown in Table A, the results of the veterinary diagnostic test on test portions from Canine B showed food sensitivities in almost all areas.

### EXAMPLE 2

Two fresh stool samples were collected from each of 2 canines approximately 2 days apart and tested.

The fresh stool samples were processed similarly to Example 1 and the testing portion was obtained from the liquid from the fresh stool sample. In some implementations, a veterinary diagnostic test that includes an absorbent portion may absorb liquid directly from the fecal sample and/or from supernatant liquid obtained after centrifuging the fecal sample.

Table B includes the results from the testing on the 2 canines. As illustrated, the day-to-day fluctuation of the result does not appear to significantly vary. Thus, the results of the veterinary diagnostic test may accurately predict food sensitivities.

**Table B: Food sensitivities results for 2 canines obtained using veterinary test**

| **Dogs Tested** | **Wheat Gluten** | **Cow's Milk** | **Chicken Eggs** | **Soy** |
|---|---|---|---|---|
| **Canine I 12/11/15** | **18** | 7 | 3 | 6 |
| **Canine I 12/13/15** | **16** | 6 | 4 | 6 |
| | | | | |
| **Canine J 12/10/15** | **15** | **25** | 4 | 7 |
| **Canine J 12/11/15** | **15** | **23** | 4 | 6 |

### EXAMPLE 3

Two fresh stool samples were collected from each of 3 canines approximately 4-5 months apart and tested.

The fresh stool samples were processed similarly to Example 1 and the testing portion was obtained from the liquid from the fresh stool sample. In some implementations, a veterinary diagnostic test that includes an absorbent portion may absorb liquid directly from the fecal sample and/or from supernatant liquid obtained after centrifuging the fecal sample.

Table C includes the results from the testing on the 3 canines. As illustrated, for Canines K and M, the veterinary diagnostic tests performed on testing portions obtained several months apart illustrate the same reactivity (e.g., positive or negative) over the time period. Thus, the month-to month fluctuation of the result does not appear to significantly vary and the results of the veterinary diagnostic test may accurately predict food sensitivities.

**Table C: Food sensitivities results for 3 canines obtained using veterinary test**

| **Dogs Tested** | **Wheat Gluten** | **Cow's Milk** | **Chicken Eggs** | **Soy** |
|---|---|---|---|---|
| **Canine K 10/1/16** | **22** | 6 | 3 | 3 |
| **Canine K 2/7/16** | **35** | 2 | 2 | 2 |
| | | | | |
| **Canine L 9/3/15** | **36** | **17** | **11** | 9 |
| **Canine L 2/4/16** | **29** | 4 | 2 | 2 |
| | | | | |
| **Canine M 10/1/2015** | **30** | 3 | 2 | 4 |
| **Canine M 2/7/2016** | **22** | 6 | 2 | 3 |

Canine L had "leaky gut" with veterinary diagnostic test results that indicated positive reactivity to several foods, such as wheat gluten, milk, egg, and even an elevated negative reactivity to soy. After the first testing, Canine L was administered a low-gluten diet, the test results from the second testing portion obtained several months later was more consistent with other canines in the study (e.g., positive reactivity for wheat gluten but not for other foods). Accordingly, identifying food sensitivities in animals may be facilitate research studies regarding for example, classes of animals, and may improve health for individual animals (e.g., by identifying food sensitivities and then eliminating and/or reducing the identified foods from the animal's diet).

### EXAMPLE 4

The results for 5 common human allergens were obtained via an implementation of a veterinary diagnostic test for the Canines in Examples 1-3. Table D includes the results from the testing on the canines. As illustrated, canines tend to have positive reactivity (e.g., food sensitivity) to wheat gluten and some canines had food sensitivities not present in other canines. For example, Canines C and J presented sensitivity to cow's milk, and Canine L, who had "leaky gut" presented sensitivity to multiple foods.

**Table D: Food sensitivities results for 13 canines obtained using veterinary test**

| **13 Dogs Tested** | **Wheat Gluten*** | **Cow's Milk*** | **Chicken Eggs*** | **Soy*** |
|---|---|---|---|---|
| **Canine A** | **12** | 5 | 3 | 3 |
| **Canine K** | **22** | 6 | 3 | 3 |
| **Canine B** | **22** | 6 | 3 | 9 |
| **Canine C** | **13** | **10** | 4 | 7 |
| **Canine D** | **16** | 7 | 2 | 3 |
| **Canine E** | **24** | 7 | 5 | 4 |
| **Canine L** | **36** | **17** | **11** | 9 |
| **Canine I** | **18** | 7 | 3 | 6 |
| **Canine M** | **30** | 3 | 2 | 4 |
| **Canine F** | **17** | 8 | 7 | 7 |
| **Canine G** | **15** | 5 | 3 | 4 |
| **Canine H** | **14** | 5 | 2 | 3 |
| **Canine J** | **15** | **25** | 4 | 7 |

### END OF EXAMPLES

Although food sensitivities have been used to describe an immunologic sensitivity to a food, food sensitivity may also refer to an immunologic sensitivity to a particular food substance. For example, food sensitivity may refer to an immunologic sensitivity to a food dye. Food sensitivity may refer to an immunologic sensitivity to a food additive. In some implementations, food sensitivity may refer to an immunologic sensitivity to a vitamin, supplement, nutraceutical, pharmaceutical, and/or other products consumed by an animal.

In various implementations, an agent has been described. An agent may include antibodies, antigens, cells, enzymes, markers, and/or portions thereof.

In various implementations, selective coupling has been described. Selective coupling may include allowing coupling between a first agent and one or more second agent and restricting coupling between the first agent and one or more third agents. The second agents may include a single type of or multiple types of the agent. The third agent may include a single type of agent and/or multiple types of agents.

Although users have been described as a human, a user may be a person or a group of people. A user may administer the test on a dog.

When testing for food sensitivities, an organism or set of organism specific antibody may be identified. For example, immunological sensitivity may produce IgA like immunoglobulin. Thus, the veterinary diagnostic tests may be utilized to determine the presence of IgA like immunoglobulin in samples (e.g., fecal, saliva, etc.).

It is to be understood the implementations are not limited to particular systems or processes described which may, of course, vary. It is also to be understood that the terminology used herein is for the purpose of describing particular implementations only, and is not intended to be limiting. As used in this specification, the singular forms "a", "an" and "the" include plural referents unless the content clearly indicates otherwise. Thus, for example, reference to "a food" includes a combination of two or more foods and reference to "a test" includes different types and/or combinations of tests.

## Claims

1. A method to diagnose immunologic food sensitivity in a dog, the method comprising the steps of:
(i) determining whether a testing portion of a fecal sample obtained from a dog includes at least one of IgA associated with a set of food sensitivities or IgA antibody associated with a set of food sensitivities, wherein the testing portion comprises a portion of the fecal sample; and
determining that one or more food sensitivities of a set of immunologic food sensitivities is present in the dog if a determination is made that at least one of IgA associated with a set of food sensitivities or IgA antibody associated with a set of food sensitivities is present in the testing portion of the fecal sample of the dog.

2. The method of claim 1 further comprising concentrating the fecal sample prior to step (i), wherein concentrating the fecal sample comprises:
centrifuging the fecal sample; and
removing a supernatant portion from the centrifuged fecal sample, wherein
the testing portion is obtained from the supernatant portion.

3. The method of claims 1 or 2 further comprising:
freeze-drying the fecal sample to a solid fecal material; and
reconstituting the solid fecal material with water to form a reconstituted fecal sample, wherein the testing portion is obtained from the reconstituted solid fecal material.

4. The method of any of claims 1-3, further comprising applying a solution comprising one or more flags to the fecal sample to obtain a flagged fecal sample, wherein the testing portion is obtained from the flagged fecal sample.

5. The method of any of claims 1-4, further comprising applying a hydrating agent to the fecal sample prior to step (i).

6. The method of any of claims 1-5, further comprising determining whether a blood sample obtained from the dog includes at least one of IgA, IgE, or IgG associated with the set of food sensitivities; wherein determining whether a set of immunologic food sensitivities is present in the dog is further based on whether a determination is made that at least one of IgA, IgE, or IgG associated with the set of food sensitivities is present in a blood sample from the dog.

7. The method of any of claims 1-6, further comprising determining whether a second testing portion of the obtained fecal sample includes at least one of IgA associated with a second set of food sensitivities or IgA antibody associated with a second set of food sensitivities if a determination is made that the set of immunologic food sensitivities is present in the dog.

8. A veterinary diagnostic test kit to diagnose immunologic food sensitivity in a dog according to the method described in any of claims 1-7, wherein the kit comprises:
a fecal sample collection tool (400);
a veterinary diagnostic test to identify the presence of a first set of food sensitivities in a dog, wherein the veterinary diagnostic test comprises:
a substrate; and
a test region (240, 440), wherein the test region comprises one or more antibody binding agents coupled to the substrate, wherein at least one of the antibody binding agents is adapted to selectively couple to one or more first antibodies associated with a first set of food sensitivities when one or more of the first antibodies is present in a fecal sample obtained from a dog, wherein the veterinary diagnostic test is integrated in the collection tool, and wherein the veterinary diagnostic test is positioned on the fecal sample collection tool such that the veterinary diagnostic test contacts the fecal sample.

9. The kit of claim 8, wherein the veterinary diagnostic test comprises a test stick (200) adapted to contact the fecal sample, and wherein one or more of the first antibodies, if present in the fecal sample, is transferred to the veterinary diagnostic test by the contact.

10. The kit of claims 8 or 9 wherein the veterinary diagnostic test further comprises an absorbent region (220) adapted to transfer a testing portion of a fecal sample of an animal from the fecal sample to the test region (240) of the veterinary diagnostic test.

11. The kit of any of claims 8-10, wherein the veterinary diagnostic test further comprises one or more flags adapted to indicate coupling of one or more first antibodies in a fecal sample with one or more of the antibody binding agents of the veterinary diagnostic test, and wherein one or more of the flags indicates that at least one of the first set of food sensitivities is present in the dog associated with the fecal sample.

12. The kit of any of claims 8-11, wherein the fecal sample collection tool (400) includes an opening to allow at least a portion of fluid (250) from a fecal sample to drain from the fecal sample collection tool.

## Patentansprüche

1. Verfahren zum Diagnostizieren einer immunologischen Nahrungsmittelempfindlichkeit bei einem Hund, wobei das Verfahren die folgenden Schritte umfasst:
(i) Bestimmen, ob ein Testabschnitt einer von einem Hund erhaltenen Fäkalprobe ein einem Satz von Nahrungsmittelempfindlichkeiten zugeordnetes IgA und/oder einen einem Satz von Nahrungsmittelempfindlichkeiten zugeordneten IgA-Antikörper beinhaltet, wobei der Testabschnitt einen Abschnitt der Fäkalprobe umfasst; und
Bestimmen, dass eine oder mehrere Nahrungsmittelempfindlichkeiten eines Satzes von immunologischen Nahrungsmittelempfindlichkeiten bei dem Hund vorhanden sind, falls eine Bestimmung gemacht wird, dass ein einem Satz von Nahrungsmittelempfindlichkeiten zugeordnetes IgA und/oder ein einem Satz von Nahrungsmittelempfindlichkeiten zugeordneter IgA-Antikörper in dem Testabschnitt der Fäkalprobe des Hundes vorhanden ist.

2. Verfahren nach Anspruch 1, ferner umfassend das Konzentrieren der Fäkalprobe vor Schritt (i), wobei das Konzentrieren der Fäkalprobe Folgendes umfasst:
Zentrifugieren der Fäkalprobe; und
Entfernen eines überstehenden Abschnitts aus der zentrifugierten Fäkalprobe, wobei der Testabschnitt aus dem überstehenden Abschnitt erhalten wird.

3. Verfahren nach Anspruch 1 oder 2, ferner Folgendes umfassend:
Gefriertrocknen der Fäkalprobe zu einem festen Fäkalmaterial; und
Rekonstituieren des festen Fäkalmaterials mit Wasser, um eine rekonstituierte Fäkalprobe auszubilden, wobei der Testabschnitt aus dem rekonstituierten festen Fäkalmaterial erhalten wird.

4. Verfahren nach einem der Ansprüche 1-3, ferner umfassend ein Aufbringen einer Lösung, die eine oder mehrere Markierungen zu der Fäkalprobe umfasst, um eine markierte Fäkalprobe zu erhalten, wobei der Testabschnitt von der markierten Fäkalprobe erhalten wird.

5. Verfahren nach einem der Ansprüche 1-4, ferner umfassend ein Aufbringen eines Hydratisierungsmittels auf die Fäkalprobe vor Schritt (i).

6. Verfahren nach einem der Ansprüche 1-5, ferner umfassend ein Bestimmen, ob eine von dem Hund erhaltene Blutprobe ein dem Satz von Nahrungsmittelempfindlichkeiten zugeordnetes IgA, IgE und/oder IgG beinhaltet; wobei das Bestimmen, ob ein Satz von immunologischen Nahrungsmittelempfindlichkeiten bei dem Hund vorhanden ist, ferner darauf basiert ist, ob eine Bestimmung gemacht wird, dass ein dem Satz von Lebensmittelempfindlichkeiten zugeordnetes IgA, IgE und/oder IgG in einer Blutprobe von dem Hund vorhanden ist.

7. Verfahren nach einem der Ansprüche 1-6, ferner umfassend das Bestimmen, ob ein zweiter Testabschnitt der erhaltenen Fäkalprobe ein einem zweiten Satz von Nahrungsmittelempfindlichkeiten zugeordnetes IgA und/oder einen einem zweiten Satz von Nahrungsmittelempfindlichkeiten zugeordneten IgA-Antikörper beinhaltet, falls eine Bestimmung gemacht wird, dass der Satz von immunologischen Nahrungsmittelempfindlichkeiten bei dem Hund vorhanden ist.

8. Veterinärmedizinisches Diagnosetestkit zum Diagnostizieren einer immunologischen Nahrungsmittelempfindlichkeit bei einem Hund gemäß dem Verfahren nach einem der Ansprüche 1-7, wobei das Kit Folgendes umfasst:
ein Fäkalprobensammelwerkzeug (400);
einen veterinärmedizinischen Diagnosetest zum Identifizieren des Vorhandenseins eines ersten Satzes von Nahrungsmittelempfindlichkeiten bei einem Hund, wobei der veterinärmedizinische Diagnosetest Folgendes umfasst:
ein Substrat; und
einen Testbereich (240, 440),
wobei der Testbereich ein oder mehrere an das Substrat gekoppelte Antikörperbindemittel umfasst, wobei wenigstens eines der Antikörperbindemittel geeignet ist, selektiv an einen oder mehrere erste einem ersten Satz von Nahrungsmittelempfindlichkeiten zugeordnete Antikörper zu koppeln, wenn einer oder mehrere der ersten Antikörper in einer von einem Hund erhaltenen Fäkalprobe vorhanden sind, wobei der veterinärmedizinische Diagnosetest in dem Sammelwerkzeug integriert ist und wobei der veterinärmedizinische Diagnosetest auf dem Fäkalprobensammelwerkzeug derart positioniert ist, dass der veterinärmedizinische Diagnosetest die Fäkalprobe berührt.

9. Kit nach Anspruch 8, wobei der veterinärmedizinische Diagnosetest einen Teststab (200) umfasst, der zu dem Berühren der Fäkalprobe geeignet ist, und wobei einer oder mehrere der ersten Antikörper, falls in der Fäkalprobe vorhanden, durch die Berührung an den veterinärmedizinischen Diagnosetest übertragen werden.

10. Kit nach Anspruch 8 oder 9, wobei der veterinärmedizinische Diagnosetest ferner einen absorbierenden Bereich (220) umfasst, der geeignet ist, einen Testabschnitt einer Fäkalprobe eines Tieres aus der Fäkalprobe an den Testbereich (240) des veterinärmedizinischen Diagnosetests zu übertragen.

11. Kit nach einem der Ansprüche 8-10, wobei der veterinärmedizinische Diagnosetest ferner eine oder mehrere Markierungen umfasst, die geeignet sind, die Kopplung eines oder mehrerer erster Antikörper in einer Fäkalprobe mit einem oder mehreren der Antikörperbindemittel des veterinärmedizinischen Diagnosetests anzuzeigen, und wobei eine oder mehrere der Markierungen anzeigen, dass wenigstens eine des ersten Satzes von Nahrungsmittelempfindlichkeiten bei dem der Fäkalprobe zugeordneten Hund vorhanden ist.

12. Kit nach einem der Ansprüche 8-11, wobei das Fäkalprobensammelwerkzeug (400) eine Öffnung beinhaltet, um wenigstens einem Abschnitt eines Fluids (250) aus einer Fäkalprobe zu ermöglichen, aus dem Fäkalprobensammelwerkzeug abzufließen.

## Revendications

1. Procédé pour diagnostiquer une intolérance alimentaire immunologique chez un chien, le procédé comprenant les étapes consistant à :
(i) déterminer si une partie de test d'un échantillon fécal obtenu d'un chien comprend au moins l'une des IgA associées à un ensemble d'intolérances alimentaires ou un anticorps IgA associé à un ensemble d'intolérances alimentaires, la partie de test comprenant une partie de l'échantillon fécal ; et
déterminer qu'une ou plusieurs intolérances alimentaires d'un ensemble d'intolérances alimentaires immunologiques est présente chez le chien s'il est déterminé qu'au moins une des IgA associées à un ensemble d'intolérances alimentaires ou qu'un anticorps IgA associé à un ensemble d'intolérances alimentaires est présent dans la partie de test de l'échantillon fécal du chien.

2. Procédé selon la revendication 1, comprenant en outre la concentration de l'échantillon fécal avant l'étape (i), la concentration de l'échantillon fécal comprenant :
la centrifugation de l'échantillon fécal ; et
le retrait d'une partie surnageante de l'échantillon fécal centrifugé,
la partie de test étant obtenue à partir de la partie surnageante.

3. Procédé selon la revendication 1 ou 2, comprenant en outre :
la lyophilisation de l'échantillon fécal en une matière fécale solide ; et
la reconstitution de la matière fécale solide avec de l'eau pour former un échantillon fécal reconstitué, la partie de test étant obtenue à partir de la matière fécale solide reconstituée.

4. Procédé selon l'une quelconque des revendications 1 à 3, comprenant en outre l'application d'une solution comprenant un ou plusieurs marqueurs à l'échantillon fécal pour obtenir un échantillon fécal marqué, la partie de test étant obtenue à partir de l'échantillon fécal marqué.

5. Procédé selon l'une quelconque des revendications 1 à 4, comprenant en outre l'application d'un agent hydratant à l'échantillon fécal avant l'étape (i).

6. Procédé selon l'une quelconque des revendications 1 à 5, comprenant en outre à déterminer si un échantillon sanguin obtenu du chien comprend au moins l'une des IgA, IgE ou IgG associées à l'ensemble d'intolérances alimentaires ; dans lequel la façon de déterminer si un ensemble d'intolérances alimentaires immunologiques est présent chez le chien est en outre basé sur la détermination qu'au moins une des IgA, IgE ou IgG associées à l'ensemble d'intolérances alimentaires est présente ou non dans un échantillon sanguin du chien.

7. Procédé selon l'une quelconque des revendications 1 à 6, comprenant en outre à déterminer si une seconde partie de test de l'échantillon fécal obtenu comprend au moins une des IgA associées à un second ensemble d'intolérances alimentaires ou un anticorps IgA associé à un second ensemble d'intolérances alimentaires s'il est déterminé que l'ensemble d'intolérances alimentaires immunologiques est présent chez le chien.

8. Kit de test de diagnostic vétérinaire pour diagnostiquer l'intolérance alimentaire immunologique chez un chien selon le procédé décrit dans l'une quelconque des revendications 1 à 7, le kit comprenant :
un outil de collecte d'échantillon fécal (400) ;
un test de diagnostic vétérinaire pour identifier la présence d'un premier ensemble d'intolérances alimentaires chez un chien, le test de diagnostic vétérinaire comprenant :
un substrat ; et
une zone de test (240, 440), la zone de test comprenant un ou plusieurs agents de liaison d'anticorps couplés au substrat, au moins un des agents de liaison d'anticorps étant conçu pour se coupler sélectivement à un ou plusieurs premiers anticorps associés à un premier ensemble d'intolérances alimentaires lorsqu'un ou plusieurs des premiers anticorps est présent dans un échantillon fécal obtenu chez un chien, le test de diagnostic vétérinaire étant intégré à l'outil de collecte, le test de diagnostic vétérinaire étant positionné sur l'outil de collecte d'échantillon fécal de telle sorte que le test de diagnostic vétérinaire entre en contact avec l'échantillon fécal.

9. Kit selon la revendication 8, dans lequel le test de diagnostic vétérinaire comprend une bandelette de test (200) conçue pour entrer en contact avec l'échantillon fécal, et dans lequel un ou plusieurs des premiers anticorps, si présent dans l'échantillon fécal, est transféré au test de diagnostic vétérinaire par le contact.

10. Kit selon la revendication 8 ou 9, dans lequel le test de diagnostic vétérinaire comprend en outre une zone absorbante (220) conçue pour transférer une partie de test d'un échantillon fécal d'un animal de l'échantillon fécal à la zone de test (240) du test de diagnostic vétérinaire.

11. Kit selon l'une quelconque des revendications 8 à 10, dans lequel le test de diagnostic vétérinaire comprend en outre un ou plusieurs marqueurs conçus pour indiquer le couplage d'un ou plusieurs premiers anticorps dans un échantillon fécal avec un ou plusieurs des agents de liaison des anticorps du test de diagnostic vétérinaire, et un ou plusieurs des marqueurs indiquant qu'au moins un des premiers ensembles de d'intolérances alimentaires est présent chez le chien associé à l'échantillon fécal.

12. Kit selon l'une quelconque des revendications 8 à 11, dans lequel l'outil de collecte d'échantillon fécal (400) comprend une ouverture pour permettre à au moins une partie du fluide (250) provenant d'un échantillon fécal de s'écouler à partir de l'outil de collecte d'échantillon fécal.
